**Europäisches Patentamt**

**European Patent Office**

**Office européen des brevets**

(19)

(11) Veröffentlichungsnummer : **0 106 269 B2**

# (12) NEUE EUROPÄISCHE PATENTSCHRIFT

(45) Veröffentlichungstag der neuen Patentschrift :
**10.03.93 Patentblatt 93/10**

(51) Int. Cl.$^5$ : **A61K 35/16**

(21) Anmeldenummer : **83109936.1**

(22) Anmeldetag : **05.10.83**

(54) **Verfahren zur Pasteurisierung von antihämophilem Kryopräzipitat (AHK) und danach hergestelltes antihämophiles Kryopräzipitat.**

Die Akte enthält technische Angaben, die nach dem Eingang der Anmeldung eingereicht wurden und die nicht in dieser Patentschrift enthalten sind.

(30) Priorität : **09.10.82 DE 3237512**

(43) Veröffentlichungstag der Anmeldung :
**25.04.84 Patentblatt 84/17**

(45) Bekanntmachung des Hinweises auf die Patenterteilung :
**08.02.89 Patentblatt 89/06**

(45) Bekanntmachung des Hinweises auf die Entscheidung über den Einspruch :
**10.03.93 Patentblatt 93/10**

(84) Benannte Vertragsstaaten :
**AT BE CH DE FR GB IT LI LU NL SE**

(56) Entgegenhaltungen :
**EP-A- 0 018 561**
**EP-A- 0 035 204**
**EP-A- 0 053 046**
**EP-A- 0 053 338**
**DE-A- 2 715 832**

(56) Entgegenhaltungen :
**Haemostasis 1, pp. 204-209.(1972/73).Ly and Godal.**
**Deutsche Medizinische Wochenschrift (1970), vol. 95 pp. 2467- 2469**
**Godal. "The effect of EDTA...". Oslo university press, Oslo 1960**

(73) Patentinhaber : **BEHRINGWERKE Aktiengesellschaft**
**Postfach 1140**
**W-3550 Marburg 1 (DE)**

(72) Erfinder : **Heimburger, Norbert, Prof. Dr.**
**Sonnenhang 10**
**W-3550 Marburg (DE)**
Erfinder : **Kumpe, Gerhardt**
**Aspherfeld 14**
**W-3552 Wetter (DE)**
Erfinder : **Wormsbächer, Wilfried**
**Blumenweg 9**
**W-3575 Kirchhain (DE)**
Erfinder : **Preis, Hans Martin**
**Am Wäldchen**
**W-3550 Marburg 16 (DE)**

(74) Vertreter : **Fischer, Hans-Jürgen, Dr. et al**
**Hoechst AG Zentrale Patentabteilung**
**Postfach 80 03 20**
**W-6230 Frankfurt am Main 80 (DE)**

EP 0 106 269 B2

## Beschreibung

Die Erfindung betrifft ein Verfahren zur Pasteurisierung von antihämophiles Kryopräzipitat (AHK), sowie ein danach hergestelltes antihämophiles Kryopräzipitat.

Zur Behandlung von Patienten mit Hämophilie A und solchen mit von Willebrand-Syndrom (vW-Syndrom) werden F VIII/vW-Konzentrate aus Humanplasma verwendet. Die Behandlung erfolgt heute nicht nur prophylaktisch, sondern zum Teil in Selbsttherapie. Dies sowie die lebenslängliche Behandlungsdauer der Patienten erfordern gut verträgliche Produkte. Das sind erfahrungsgemäss die am höchst gereinigten, denn der Gehalt an Ballastproteinen erschwert nicht nur die Auflösung und Anwendung solcher Konzentrate, sondern kann zu einer Sensibilisierung gegen Fremdproteine führen, wobei die damit verbundenen Nebenreaktionen, speziell wenn sie während der Selbsttherapie auftreten, ein grosses Risiko für den Patienten darstellen.

Nomenklatur des Faktor VIII-Moleküls

F VIII assoz.Ag  =  F VIII AG  =  F VIII R:AG (related
Antigen)
F VIII v.Willebrand Faktor  =  F VIII vWF  =  F VIII R:CoF
(Ristocetin Co-Faktor)
F VIII Aktivität  =  F VIII C  (coagulation)  =  F VIII C:AG
(Coagulation Antigen)*

* Da die Aktivität durch homologe Antikörper gehemmt werden kann, spricht man hier ebenfalls von einem C-Antigen. Die eingerahmten Nomenklaturen werden international angewandt.

Für die Behandlung der Hämophilie A gibt es F VIII-Konzentrate mit hoher Reinheit, guter Wirkung und Verträglichkeit. Aus der deutschen Offenlegungsschrift 2 916 711 ist auch eine hepatitissicheres F VIII-Konzentrat bekannt.

Überraschenderweise zeigen jedoch F VIII-Konzentrate keinen optimalen therapeutischen Effekt bei der Behandlung des vW-Syndroms, obwohl sie nach in vitro-Testung neben F VIII:C auch F VIII R:AG und F VIII R:CoF enthalten. Offenbar korreliert der in vitro Test, der für die Bestimmung des vW-Syndroms verwendet wird, der Ristocetin-Cofaktor-Test, nicht mit der Blutungszeit.

Dagegen besitzt antihämophiles Kryopräzipitat einen guten therapeutischen Effekt bei vW-Syndrom Aufgabe der Erfindung war es deshalb. ein pasteurisiertes und somit hepatitis-sicheres AHK mit guter F VIII- und vW-Aktivität herzustellen.

In der EP-A-0 053 338 ist ein Verfahren zur Herstellung einer praktisch hepatitis-sicheren Präparation der Blutgerinnungsfaktoren IX und/oder X durch Erwärmen beschrieben, wobei gegebenenfalls in Gegenwart einer Aminosäure und/oder eines Saccharids oder Zuckeralkohols in Gegenwart von Calciumionen erwärmt wird.

Albumin gilt als hepatitissicher, wenn es in wässriger Lösung auf 60 °C erhitzt wird (Gellis, S. S. et al., J. Clin. Invest. (1948) 27, 239). Es wird deshalb angenommen, dass ein in Gegenwart geeigneter Stabilisatoren erhitztes Faktor IX- und/ oder X-Konzentrat ebenfalls hepatitissicher ist.

In der deutschen Offenlegungsschrift 2 916 711 ist weiterhin ein Verfahren zur Stabilisierung anderer Gerinnungsfaktoren in wässriger Lösung gegen Wärme durch Zusatz einer Aminosäure und eines Mono- oder Oligosaccharids oder Zuckeralkohols beschrieben.

Aus Haemostasis 1, 204-209 (1972/1973) ist bekannt, daß Calcium in Lösungen von gereinigtem Fibrinogen von geringer Konzentration (0.33%) einen schützenden Einfluß gegen eine mäßige Denaturierung unter dem Einfluß von Alkali oder Wärme ausübt, wobei als Test eine 40-minütige Erhitzung auf 47°C dient.

Für gewerbliche Zwecke gebrauchte Lösungen von Kryopräzipitat haben jedoch eine um etwa den Faktor 5 größere Konzentration von Fibrinogen (etwa 2% und mehr) und enthalten neben Fibrinogen beispielsweise auch schwerlösliches Fibronectin, die bei einer zur Virusinaktivierung für erforderlich gehaltenen Erhitzung für 10 Stunden auf 60° ausfallen.

Die Aufgabe wurde im Prinzip wie folgt gelöst: eine AHK-Lösung wird in Gegenwart von Ca-Ionen, einer Aminosäure und eines Mono- oder Oligosaccharids oder Zuckeralkohols, wobei das Saccharid oder der Zuckeralkohol vor der Aminosäure zugegeben wird, erhitzt.

Die Citrat-Konzentration sollte kleiner als 10 mmol/l sein und ein Zusatz von Ca-Ionen ist vorteilhaft; andernfalls tritt während der Pasteurisierung eine Gelierung auf.

Gegenstand der Erfindung ist deshalb ein Verfahren zur Pasteurisierung von antihämophilem Kryopräzipitat (AHK), dadurch gekennzeichnet, dass eine Lösung von AHK in Gegenwart von Ca-Ionen, einer Aminosäure und eines Mono- oder Oligosaccharids oder Zuckeralkohols 5-15 Stunden auf 50-70 °C erhitzt wird.

Ca-Ionen sollten in einer Konzentration von mindestens 0,2 mmol/l und höchstens 100 mmol/l, vorzugsweise 5 mmol/l, enthalten sein. Sie werden bevorzugt in Form einer $CaCl_2$-Lösung zugeführt.

Bevorzugt wird mindestens eine der Aminosäuren Glycin, $\alpha$- oder $\beta$-Alanin, Hydroxyprolin, Prolin, Glutamin $\alpha$- $\beta$- oder $\gamma$-Aminobuttersäure, vorzugsweise Glycin, zugesetzt. Die Konzentration beträgt 1-3 mol/l.

Als Kohlenhydrat wird vorzugsweise Saccharose in einer Konzentration von 35-60 g/100 ml Lösung verwendet. Für eine genügende Stabilisierung bei der Pasteurisierung von AHK sind Saccharose-Konzentrationen von über 30 g/100 ml Lösung erforderlich vorzugsweise 60 g/100 ml, und Glycin-Konzentrationen von 2 mol/l; bei geringeren Konzentrationen bilden sich Coagula während des Erhitzens und die F VIII:C-Aktivität fällt ab (bei weniger als 2 mol/l Glycin).

Weiterhin sollte die Konzentration von Komplexbildnern, wie sie für die Blutentnahme verwendet werden, unter 10 mM Citrat und unter 5 mM EDTA liegen. Die Beobachtung, das Fibrinogen, das frei von Komplexbildnern ist, beim Erhitzen nicht coaguliert und klar bleibt, erlaubt die Schlussfolgerung dass die Denaturierung auf Entzug der Ca-Ionen zurückzuführen ist. Offenbar bindet das Fibrinogen das Calcium so fest, dass Komplexbildner in niedrigen Konzentrationen es ihm nicht zu entziehen vermögen Diesbezüglich ist von bedeutung dass bei gleichen Stabilisatoren die Erhitzung in Gegenwart von 5 mmol Citrat eine klare AHK-Lösung ergibt, während sich mit 5 mmol EDTA ein Coagulum bildet.

Eine exakte Ca-Ionen-Konzentration ist für dieses heterogene Gemisch nicht anzugeben; denn neben dem Faktor VIII wird auch das Fibrinogen, das den Hauptanteil dieser Fraktion darstellt, durch $Ca^{2+}$ stabilisiert. Am besten sollten Komplexbildner abwesend, aber Ca-Ionen in einer Konzentration von 0,2-100 mmol/l, vorzugsweise 5 mmol/l, anwesend sein (Tabelle 1).

Die Erhitzung wird solange durchgeführt, bis in dem AHK möglicherweise vorhandenes infektiöses Hepatitis B-Virus seine Infektiosität veriiert. Dazu wird 1 Minute bis 48 Stunden bei einer Temperatur zwischen 30 und 80 °C, vorzugsweise 5-15 Stunden, bei einer Temperatur von 50 bis 70°C erhitzt.

Nach Untersuchungen in der Polyacrylamid-Gelelektrophorese ist das erfindungsgemäss erhältliche AHK frei von Fibrinogen-Polymeren; aufgrund seines hohen Gehalts an Fibrinogen, F VIII:C, F VIII R:CoF und Fibronectin ist ein optimales Therapeutikum für die Behandlung von Hämophilien und des vW-Syndroms, im besonderen deswegen, weil es durch die Pasteurisierung als hepatitis-sicher gilt. Ein weiterer Vorteil liegt in der im Vergleich zu anderen Kryo-Lösungen guten Löslichkeit des Präparates, die auf die Abtrennung von schwerlöslichen Proteinen und Polymeren speziell des Fibrinogens, zurückzuführen ist.

Die erhitzte Lösung wird mit einer Pufferlösung verdünnt und Begleit- und Denaturierungs-Proteine durch eine Vorfällung mit Glycin abgetrennt, bevor das AHK durch Aufsättigung der Glycin-Konzentration auf 2,2 mol/l und Kochsalz (12 g/ 100 ml) präzipitiert wird. Der Fällungsrückstand wird durch Zentrifugation gewonnen. gelöst, dialysiert, die Faktor VIII-Aktivität bestimmt und eingestellt auf 6-8 E/ml; nach Klär- und Sterilfiltration wird die Lösung in 100 ml-Mengen in 250 ml Infusionsflaschen abgefüllt und lyophilisiert.

Die nachfolgend tabellarisch zusammengestellten Versuche wurden durchgeführt:

Tabelle 1

Hitzestabilität von AHK mit verschiedenen Zusätzen zur Stabilisierung

| Saccharose (g/100 ml) | Glycin (mol) | Citrat (mmol) | EDTA (mmol) | Ca²⁺ (mmol) | nach 10 h bei 60°C |
|---|---|---|---|---|---|
| 15 | 2 | 5 | – | – | Ausfäll.* + Coagulation |
| 30 | 2 | 5 | – | – | coaguliert |
| 60 | 2 | 5 | – | – | klar |
| 60 | – | 5 | – | – | coaguliert |
| 60 | 0,5 | 5 | – | – | fast klar |
| 60 | 1 | 5 | – | – | klar |
| 60 | 2** | 5 | – | – | klar |
| 60 | 2 | 5 | – | – | klar |
| 60 | 2 | 10 | – | – | coaguliert |
| 60 | 2 | 20 | – | – | coaguliert |
| 60 | 2 | – | 5 | – | coaguliert |
| 60 | 2 | – | 10 | – | coaguliert |
| 60 | 2 | – | – | 2,5 | klar |
| 60 | 2 | – | – | 5 | klar |
| 60 | 2 | – | – | 10 | klar |
| 60 | 2 | – | – | 25 | klar |

\* Saccharose-Konzentration reicht nicht aus, um das Fibrinogen in Lösung zu halten.
\*\* Diese Glycin-Konzentration ist notwendig, um die Faktor VIII:C-Aktivität während der Erhitzung zu konservieren.

Tabelle 2 enthält die Ergebnisse der Aktivitätsprüfung von AHK aus 60 ml unbehandeltem Kryopräzipitat nach 10-stündigem Erhitzen auf 60 °C.

Zur Abtrennung der während der Erhitzung entstandenen Denaturierungsprodukte kann eine 1,3 mol/l Glycinfällung und zur Entfernung der Stabilisatoren und Konzentrierung des F VIII/vW-Proteins eine 2,2 mol/l Fällung mit Glycin und 12 g NaCl/100 ml Lösung durchgeführt werden. Die Erfindung wird am folgenden Beispiel näher erläutert.

Tabelle 2

| Zusätze Saccharose (g/100 ml) | Glycin (mol) | Citrat (mmol) | Ca⁺⁺ (mmol) | F VIII:C Rest-aktivität nach Erhitzen (%) | Volu-men (ml) | OD₂₈₀ | Aktivitäten nach Erhitzen* | | |
|---|---|---|---|---|---|---|---|---|---|
| | | | | | | | F VIII:C | F VIII R:AG | F VIII/vW |
| 60 | 2 | – | – | 96 | 38 | 14,1 | 5,6 | 20 | 10 |
| 60 | 2 | – | – | 95 | 40 | 13,0 | 8,8 | 24 | 14 |
| 60 | 2 | – | 2,5 | 90 | 42 | 14,5 | 4,8 | 17 | 8,6 |
| 60 | 2 | – | 5 | 86 | 45 | 16 | 8,0 | 32 | 14 |
| 60 | 2 | – | 10 | 88 | 64 | 10 | 5,6 | 17 | 9,8 |
| 60 | 2 | 5 | – | 96 | 40 | 12,9 | 4,8 | 20 | 10 |

\* In Einheiten pro ml bezogen auf die Aktivität von 1 ml Citratplasma (= 1E)
Testverfahren für F VIII:C: Einphasenmethode nach Simone et al.; J. Lab. Clin. Med. 69, 706 (1967)
F VIIIR:AG: Elektroimmundiffusion nach Laurell; Analyt. Biochem. 15, 45 (1966)
F VIII:vW: Plättchenagglutinationstest mit von Willebrand-Faktor-Reagenz Behringwerke, Rivard et al.; Thromb. Res. 12, 677 (1978)

Beispiel:

Herstellung von pasteurisiertem AHK

Ausgangsmaterial: 250 g unbehandeltes Kryopräzipitat (Kryo), wie es nach Separatortrennung aus Citratplasma anfällt (G Pool: Cryoprecipitate: its Preparation and Clinical Use; Handbook of Hemophilia; ed. by K.M. Brinkhous and H.C. Hemker, Part II, 1975).

I Al(OH)$_3$-Adsorption

250 g Kryo wurden bei 37°C in soviel 0,1 mol/l NaCl-Lösung gelöst, dass 1 l Lösung entstand, mit 80 ml einer Suspension von 1 g Al(OH)$_3$, in 100 ml Wasser versetzt und 15 Minuten gerührt. Das Al(OH)$_3$ wurde durch Zentrifugation abgetrennt und verworfen.

II Stabilisieren und Pasteurisieren

Zu 1000 ml Kryo-Lösung aus I. wurde soviel einer wässrigen CaCl$_2$-Lösung gegeben, dass die Mischung 5 mmol/l Ca$^{++}$ enthielt, und bei 37°C mit 1000 g Saccharose versetzt. Sobald die Saccharose gelöst war, wurden 150 g Glycin zugefügt und aufgelöst.

Der pH-Wert wurde mit 2 n NaOH auf 7,3 eingestellt und die Lösung anschliessend im Wasserbad 10 Stunden lang auf 60°C erhitzt.

Isolierung der Proteine

III Abtrennung von Begleitproteinen und Denaturierungsprodukten

Die unter II. erhaltene Lösung wurde nach Erkalten mit 5 l Citrat-NaCl Puffer (0,06 mol/l NaCl und 0,02 mol/l Tri-Na-Citrat) verdünnt, unter Rühren bei 37°C mit 648 g Glycin versetzt; nach 15 Minuten wurde auf 15 °C abgekühlt und zentrifugiert. Der Rückstand wurde verworfen.

IV Gewinnung des F VIII/vW-Komplexes

Der Überstand aus III. wurde unter Rühren bei 37 °C mit 449 g Glycin versetzt und anschliessend 798 g NaCl eingerührt, so dass die NaCl-Konzentration 12 g/100 ml betrug. Nach Lösen aller Zusätze wurde auf 15 °C abgekühlt. Durch Zentrifugation bei 3000 g wurde eine klare Trennung der Fällung von Überstand erzielt. Der Rückstand wurde in 200 ml Puffer (0,06 mol/l NaCl, 0,02 mol/l Tri-Na-Citrat, pH 7,3 und 1 g Glycin/100 ml) aufgelöst. Es wurden 350 ml Lösung erhalten.

V Dialyse

Die Lösung wurde gegen 20 l des gleichen Puffers wie unter IV. dialysiert, was zu 400 ml Lösung mit einer Leitfähigkeit von 14 mS führte.

VI Endkonfektionierung

Dazu wurde eine Ultrazentrifugation, eine Klärfiltration sowie eine Sterilfiltration angeschlossen Ausbeute: Es wurden 500 ml pasteurisierte AHK-Lösung erhalten, die gegebenenfalls lyophilisiert wurden.

Es folgt die Charakterisierung von drei Chargen pasteurisiertem AHK, die nach dem im Beispiel beschriebenen Verfahren hergestellt wurden:

Aktivitäten nach Lösen von 1720 mg Lyophilisat in 50 ml destilliertem Wasser.

| Charge | F VIII:C  F VIII R:AG  F VIII R:CoF (Einheiten pro ml, bezogen auf Aktivität in 1 ml Citratplasma) | | | Fibri-nogen (mg/ 100 ml) | Fibro-nectin | Fibrinogen n. Blombäck Prozent gerinnbares Eiweiss |
|---|---|---|---|---|---|---|
| 1 | 7 | 31 | 12 | 863 | 193 | 79 |
| 2 | 6,8 | 27 | 20 | 1189 | 218 | 79 |
| 3 | 8,8 | 29 | 15 | 1243 | 246 | 79 |

Die Aktivität an F VIII:C liegt bei allen drei Chargen in der Grössenordnung von etwa 35% des theoretischen Wertes bezogen auf das als Kryo verwendete Ausgangsmaterial; der entsprechende F VIII R:CoF-Wert liegt bei 100%.

**Patentansprüche**

1. Verfahren zur Pasteurisierung von antihämophilem Kryopräzipitat (AHK), dadurch gekennzeichnet, daß eine Lösung von AHK in Gegenwart von Ca-Ionen, einer Aminosäure und eines Mono- oder Oligosaccharids oder Zuckeralkohols für eine Zeit von 5 bis 15 Stunden auf eine Temperatur von 50 bis 70°C erhitzt wird.

2. Verfahren nach Anspruch 1, dadurch gekennzeichnet, daß Ca-Ionen in einer Konzentration von 0,2-100 mmol/l vorliegen.

3. Verfahren nach Anspruch 1, dadurch gekennzeichnet, daß die Aminosäure Glycin, α- oder β-Alanin, Hydroxyprolin, Prolin, Glutamin, α-, β- oder gamma-Aminobuttersäure in einer Konzentration von 1 - 3 mol/l ist.

4. Verfahren nach Anspruch 1, dadurch gekennzeichnet, daß das Kohlenhydrat Saccharose in einer Konzentration von 35 - 60 g/100 ml Lösung ist.

5. Antihämophiles Kryopräzipitat hergestellt nach einem der Ansprüche 1 - 4.


**Claims**

1. A process for the pasteurization of antihemophilic cryoprecipitate (AHC), which comprises heating a solution of AHC in the presence of Ca ions, of an amino acid and of a mono- or oligosaccharide or sugar alcohol for a period of 5 to 15 hours at a temperature of 50 to 70°C.

2. The process as claimed in claim 1, wherein Ca ions are present at a concentration of 0.2 - 100 mmol/l.

3. The process as claimed in claim 1, wherein the amino acid is glycine, α- or β-alanine, hydroxyproline, proline, glutamine, α-, β- or gamma-aminobutyric acid in a concentration of 1-3 mol/l.

4. The process as claimed in claim 1, wherein the carbohydrate is sucrose in a concentration of 35 - 60 g/ 100 ml of solution.

5. Antihemophilic cryoprecipitate prepared as claimed in one of claims 1 - 4.


**Revendications**

1. Procédé de pasteurisation d'un cryoprécipité antihémophilique (CAH), caractérisé en ce qu'on chauffe une solution de CAH en présence d'ions Ca, d'un aminoacide et d'un mono- ou oligosaccharide ou d'un alcool de sucre pendant 5 à 15 heures à une température de 50 à 70°C.

2. Procédé suivant la revendication 1, caractérisé en ce que les ions Ca sont à une concentration de 0,2 à 100 mmoles/l.

3. Procédé suivant la revendication 1, caractérisé en ce que l'aminoacide est la glycine, l'α- ou la β-alanine, l'hydroxyproline, la proline, la glutamine, l'acide α-, β- ou γ-aminobutyrique à une concentration de 1 à 3 moles/l.

4. Procédé suivant la revendication 1, caractérisé en ce que l'hydrate de carbone saccharose est à une concentration de 35-60 g/100 ml de solution.

5. Cryoprécipité antihémophilique préparé suivant l'une des revendications 1 à 4.